## Europäisches Patentamt

## European Patent Office

## Office européen des brevets

(19)

(11) Veröffentlichungsnummer : **0 041 704**
**B1**

(12) **EUROPÄISCHE PATENTSCHRIFT**

(45) Veröffentlichungstag der Patentschrift :
09.01.85

(21) Anmeldenummer : 81104322.3

(22) Anmeldetag : 04.06.81

(51) Int. Cl.⁴ : **G 01 N 33/96, G 01 N 33/92,
G 01 N 33/68, C 12 Q 1/00**

(54) **Verfahren und Mittel zur Auflösung von Chylomikronen in wässrigem Medium.**

(30) Priorität : 06.06.80 DE 3021457

(43) Veröffentlichungstag der Anmeldung :
16.12.81 Patentblatt 81/50

(45) Bekanntmachung des Hinweises auf die Patenterteilung : 09.01.85 Patentblatt 85/02

(84) Benannte Vertragsstaaten :
**AT BE CH DE FR GB IT LI LU NL SE**

(56) Entgegenhaltungen :
EP-A- 0 009 596
DE-A- 2 011 352
DE-A- 2 724 757
US-A- 3 062 623
US-A- 3 627 468
US-A- 4 110 077

(73) Patentinhaber : **BOEHRINGER MANNHEIM GMBH
Patentabteilung, Abt. E Sandhofer Strasse 112-132
Postfach 31 01 20
D-6800 Mannheim 31 Waldhof (DE)**

(72) Erfinder : **Batz, Hans-Georg, Dr.
Traubinger Strasse 63
D-8132 Tutzing (DE)**
Erfinder : **Looser, Siegfried, Dr.
Ammerstrasse 27
D-8120 Weilheim (DE)**

(74) Vertreter : **Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)**

## Beschreibung

Die Erfindung betrifft ein Verfahren und ein Mittel zur Auflösung von Chylomikronen in wäßrigen Medien, wie z. B. trüben Seren, unter Verwendung eines oberflächenaktiven Polyäthylenglykol-Derivats.

Chylomikronen sind feine Teilchen, die zu über 90 % aus Lipiden und Cholesterin bestehen und eine Hülle aus Lipoproteinen aufweisen. Sie kommen in biologischem Material vor, vor allem in Blut bzw. Serum. Durch derartige Chylomikronen getrübte Seren werden vor allem dann erhalten, wenn die Blutentnahme für die Serumgewinnung nach einer fettreichen Nahrungsaufnahme erfolgt oder bei bestimmten Krankheitszuständen. Derartige, durch Chylomikronen getrübte Seren lassen sich nicht zu Bestimmungen verwenden, bei denen photometrische Messungen erfolgen.

Die Chylomikronenfraktion dient ferner als Ausgangsprodukt für die Gewinnung von darin enthaltenen Substanzen. Auch in diesem Fall ist es erforderlich, die Chylomikronen in wäßrigem Medium aufzulösen, damit eine schonende Gewinnung der Bestandteile möglich ist und eine homogene Umsetzung durchgeführt werden kann.

Kombinationen aus Lipiden, Phospholipiden und Lipoproteinen kommen auch in den Zellmembranen vor, jedoch in anderen prozentualen Zusammensetzungen. Es ist bereits eine Reihe von Detergentien bekannt, welche derartige Zellmembranen aufzulösen vermögen. Vor allem eignen sich hierzu nichtionische Tenside vom Typ eines Polyäthylenglykoläthers mit n-Alkanolen oder mit Alkylphenol. Diese Tenside vermögen jedoch Chylomikronen nicht aufzulösen.

Aus der DE-AS 23 27 894 ist es bereits bekannt, Trübungen mit Polyäthylenglykolestern von Lauraten, die eine bestimmte Zusammensetzung und damit einen bestimmten HLB-Wert aufweisen, aufzuhellen. Ein wesentlicher Nachteil der Lauratester besteht jedoch darin, daß die Esterbindung durch Lipasen und Esterasen, welche häufig zusammen mit den Chylomikronen in biologischem Material vorkommen, gespalten werden, so daß die Brauchbarkeit zur Auflösung von Chylomikronen beschränkt ist.

Aus Z. klin. Chem. 3 (1965) 96-99 ist es bekannt, im Rahmen einer Eisenbestimmungsmethode Chylomikronen, welche diese Bestimmung stören, durch Zusatz eines sekundäre Alkansulfate enthaltenden Netzmittels aufzulösen. Primäre Alkylsulfate, wie Natriumdodecylsulfat, erwiesen sich dagegen als für die Auflösung unbrauchbar.

Dieses Mittel ist jedoch im Handel nicht mehr erhältlich.

Aus EPA 0 009 596 ist eine wäßrige Lipid-Standardlösung bekannt, die ein nichtionisches und ein ionisches Detergens enthält, mit hohem Lösungsvermögen für Lipide, die dem Blutserum sehr ähnlich ist.

Aus US-PS 3 062 623 ist ein Verfahren und ein Reagenz zur Abtrennung von Fett bekannt, das ein nichtionisches und ein ionisches Detergens enthält.

Es besteht daher Bedarf an einem Verfahren und einem Mittel, welches Chylomikronen in wäßrigem Medium aufzulösen vermag. Insbesondere besteht Bedarf an einem derartigen Verfahren bzw. Mittel, bei welchem die Aufhellung rasch und vollständig verläuft und spätestens nach 3 bis 5 Minuten abgeschlossen ist.

Der Erfindung liegt die Aufgabe zugrunde, diesen Bedarf zu befriedigen. Gelöst wird diese Aufgabe durch ein Verfahren zur Auflösung von Chylomikronen in wäßrigem Medium durch Zusatz eines oberflächenaktiven Polyäthylenglykol-Derivats, welches dadurch gekennzeichnet ist, daß man

a) 3-20 Vol.% wenigstens eines Polyäthylenglykoläthers eines Alkanols oder Alkylarylalkohols mit verzweigter Alkankette und einem HLB-Wert von 12 bis 14 und

b) 1-10 Vol.% wenigstens eines sekundären Alkansulfonats mit 10-20 C-Atomen im Molekül, sowie gegebenenfalls

c) ein Alkali-p-toluolsulfonat

zusetzt.

Besonders gute Ergebnisse werden erhalten, wenn man den Polyäthylenglykoläther des verzweigten Alkanols und das bzw. die Alkansulfonate im Verhältnis von 8 bis 4 Gew.-Teilen Polyäthylenglykoläther und 2 bis 6 Gew.-Teilen Alkansulfonat einsetzt.

Da durch den Zusatz des verzweigten Polyäthylenglykoläthers und des Alkansulfonats eine erhebliche Zunahme der Viskosität des Wäßrigen Mediums erfolgen kann, ist es in vielen Fällen zweckmäßig, zur Erniedrigung der Viskosität außerdem noch ein Alkalitoluolsulfonat, vorzugsweise das Natriumsalz, zuzusetzen. Die zugesetzte Menge hängt ab von der gewünschten Viskositätsverminderung und der Ausgangsviskosität des wäßrigen Mediums, welches das verzweigte Polyäthylenglykoläther und Alkansulfonat enthält. Im allgemeinen erhält man eine ausreichende Viskositätsverminderung bei Zusätzen von 5 bis 10 Gew.-%, bezogen auf die Summe der oben genannten, beiden Zusätze. Beim verzweigten Polyäthylenglykoläther wird ein solcher mit einem HLB-Wert von etwa 12,5 bis etwa 13,5 bevorzugt. Die Bereiche des HLB-Werts von 12 bis 14 bzw. des bevorzugten Bereichs 12,5 bis 13,5 würden bei Polyäthylenglykoläthern geradkettiger Alkanole bzw. Alkylarylalkohole einem Mittel von 7 bis 12, vorzugsweise von 8 bis 10 Polyäthylenoxid-Einheiten entsprechen. Bei den verzweigten Alkanoläthern verschieben sich diese Zahlen etwas, jedoch nicht wesentlich. Da die technischen Polyäthylenglykoläther stets als Gemische mit unterschiedlicher Kettenlänge des Polyäthylenglykolanteils vorliegen, bedeuten die oben genannten Zahlenwerte nur Durchschnittswerte. Die Kettenlängenverteilung soll jedoch die Maximalwerte innerhalb der oben angegebenen Kettenlängen aufweisen. Die Bestimmung von HLB-Werten (hydrophilic lipophilic balance) ist bekannt, z. B. aus H. Stache « Tensid-Taschenbuch », Carl Hanser-Verlag, München, Wien 1979, Seiten 70-72.

Typische Beispiele für im Rahmen der Erfindung geeigneten Polyäthylenglykoläther von Alkylarylalkoholen mit verzweigten Alkanketten sind z. B. die Äther des tert.-Octylphenols, des Isooctylphenols, des Isononylphenols, Isodecylphenols usw. Der Alkanol- bzw. Alkylarylalkoholrest kann 4 bis etwa 20 C-Atome aufweisen. Enthält er einen Arylrest, vorzugsweise einen Phenylrest, so sollte die verzweigte Alkylkette wenigstens 7 Kohlenstoffatome aufweisen. Unter den keine Arylgruppen enthaltenden Äthern seien beispielsweise genannt solche des Isooctanols, Isononanols, Isodekanols, Isoundekanols, Isododekanols, Isotridekanols und Isotetradekanols usw. bis zu 16 C-Atomen. Bevorzugt werden die verzweigten Alkanole mit 12 bis 14 C-Atomen verwendet. Unter den bevorzugten Isotridekanolen seien beispielsweise genannt das Trimethyldekanol, Tetramethylnonanol und Pentamethyloctanol. Die zugesetzte Menge liegt zweckmäßig zwischen 3 und 20 Vol.%, vorzugsweise 4 bis 8 %, bezogen auf den Gesamtansatz.

Unter den Alkansulfonaten eignen sich allgemein die sekundären Alkyl- mono- und disulfonate mit 10 bis 20 Kohlenstoffatomen im Molekül. Bevorzugt werden solche mit 13 bis 15 Kohlkenstoffatomen im Molekül. Es können sowohl reine Verbindungen als auch Gemische verwendet werden. Bevorzugt verwendet man Gemische von sekundären Alkyl- mono- und disulfonaten mit bis zu 50 % disulfoniertem Anteil. Geeignete Gemische sind im Handel erhältlich, beispielsweise unter der Handelsbezeichnung « Mersolat ». Die zugesetzte Menge an Alkansulfonat liegt zweckmäßig zwischen 1 und 10 Vol.% des Gesamtansatzes, vorzugsweise zwischen 2 und 6 %.

Das erfindungsgemäße Verfahren läßt sich durchführen, indem man die einzelnen Bestandteile nacheinander zugibt. Zweckmäßig verwendet man jedoch eine Vermischung der verzweigten Polyäthylenglykoläther und der Alkansulfonate, welche auch bereits ein Alkalitoluolsulfonat enthalten kann und setzt diese Vormischung dem die Chylomikronen enthaltendenwäßrigen Medium in einer Menge zu, die zu einer Auflösung der Chylomikronen in kurzer Zeit führt. Die Auflösungsdauer sollte unter 5 Minuten, vorzugsweise unter 3 Minuten liegen. Durch einen Vorversuch läßt sich jeweils rasch feststellen, welche Menge an auflösenden Zusätzen erforderlich ist. Im allgemeinen liegt diese Menge bei trüben Seren zwischen etwa 4 und etwa 30 Vol.%. Erfolgt die Auflösung der Chylomikronen zu präparativen Zwecken, so tritt die Auflösungsgeschwindigkeit als wichtiger Faktor in den Hintergrund. Man wird dann eine möglichst geringe Menge zusetzen, um nicht unnötig große Verunreinigungen einzuschleppen.

Eine möglichst rasche Auflösung der Chylomikronen ist besonders dann von Bedeutung, wenn das erfindungsgemäße Verfahren im Rahmen der Bestimmung von Serumbestandteilen auf Analysenautomaten angewendet wird. In diesem Falle sind durch die Konstruktion des Automaten zumeist die Zeiten vorgegeben, innerhalb deren die Auflösung erfolgen muß und insbesondere bei Hochleitungsautomaten sind diese Zeiten sehr kurz und liegen vielfach unter einer Minute. Mit dem erfindungsgemäßen Verfahren ist es möglich, auch in solchen Fällen die vorgegebenen Zeitbedingungen zu erfüllen.

Ein weiterer Gegenstand der Erfindung ist ein Mittel zur Auflösung von Chylomikronen in wäßrigem Medium mit einem Gehalt an oberflächenaktiven Polyäthylenglykol-Derivaten, welches dadurch gekennzeichnet ist, daß es

a) 3-20 Vol.% wenigstens eines Polyäthylenglykoläthers eines Alkanols oder Alkylarylalkohols mit verzweigter Alkankette und einem HLB-Wert von 12 bis 14 und
b) 1-10 Vol.% wenigstens eines sekundären Alkansulfonats mit 10-20 Kohlenstoffatomen im Molekül sowie gegebenenfalls
c) ein Alkali-p-toluolsulfonat enthält.

Vorzugsweise enthält das Mittel 8 bis 4 Gew.-Teile Polyäthylenglykoläther auf 2 bis 6 Gew.-Teile Alkansulfonat sowie gegebenenfalls 5 bis 10 Gew.-% Alkalitoluolsulfonat, bezogen auf die Summe der beiden vorstehend erwähnten essentiellen Bestandteile. Für die allgemeinen und die bevorzugten Bereiche des HLB-Werts bzw. der Kettenlänge des Polyäthylenglykols gelten die oben beim Verfahren gemachten Ausführungen sinngemäß. Gleiches gilt für die Alkansulfonate.

Die mit dem erfindungsgemäßen Verfahren und Mittel erzielbaren technischen Wirkungen sind überraschend, da die primären und sekundären Alkansulfonate allein überhaupt keine auflösende Wirkung für Chylomikronen zeigen und die Polyäthylenglykoläther der verzweigten Alkanole ebenfalls nur eine unzureichende auflösende Wirksamkeit zeigen. Die synergistische Verstärkung der beiden essentiellen Bestandteile des erfindungsgemäß angewendeten Mittels ist daher überraschend. Dies zeigen die unten in den Beispielen und in der Zeichnung dargestellten Ergebnisse.

Das erfindungsgemäße Verfahren und Mittel eignet sich, wie bereits erwähnt, insbesondere im Rahmen von analytischen Bestimmungen von Serumbestandteilen. Beispielsweise seien hier genannt die Triglycerid-Bestimmungen mit Glycerinkinase als Indikatorsystem, mit Glycerindehydrogenase als Indikatorsystem, die Eisen-Bestimmung nach Lauber, die Cholesterin-Bestimmungen mit Cholesterinoxidase und Katalase oder mit Cholesterinoxidase, Phenol und 4-Aminophenazon.

Die folgenden Beispiele erläutern die Erfindung weiter.

**0 041 704**

Beispiele 1 bis 10

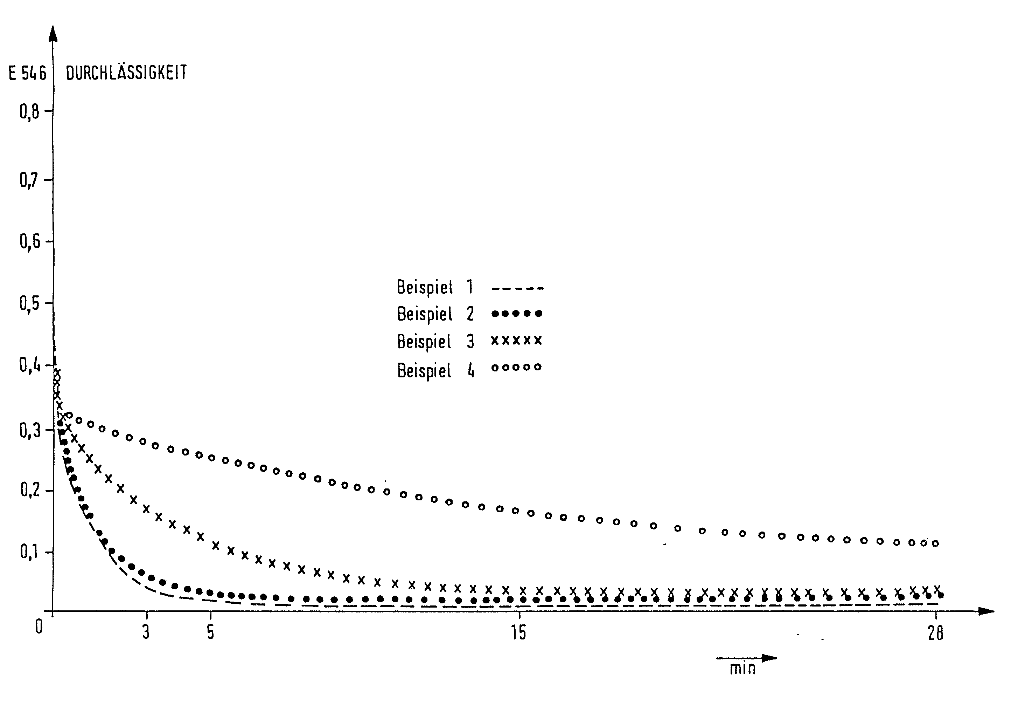

In einer 1 cm Küvette werden bei 25 °C 0,5 ml eines durch Chylomikronen stark getrübten Serums mit 1,5 ml eines Zusatzmittels der in der nachstehenden Tabelle angegebenen Zusammensetzung vermischt. Anschließend wird die Änderung der Durchlässigkeit bei 546 nm in Abhängigkeit von der Zeit bestimmt. Die erhaltenen Ergebnisse sind in Fig. 1 der Zeichnung graphisch dargestellt.

Das zugesetzte Mittel bestand aus einer wäßrigen Lösung, welche Polyäthylenglykoläther, Alkansulfonat und Natriumtoluolsulfonat in den in der nachstehenden Tabelle I angegebenen Mengen enthielt. In der Tabelle sind auch die verwendeten Polyäthylenglykoläther näher beschrieben. Als Alkansulfonat wurde eine im Handel unter der Bezeichnung « Mersolat H » erhältliche Mischung sekundärer Alkansulfonate mit 13 bis 15 C-Atomen und 40 bis 50 % sekundärem Anteil verwendet.

Tabelle I

| Beispiel | Polyäthylenglykoläther | | | | Alkansulfonat | Na-Toluolsulfonat |
| | (Aryl)-Alkanolrest | HLB-Wert | (ÄO) | Menge % | Menge % | Menge % |
|---|---|---|---|---|---|---|
| 1 | Isotridecyl | 12 | | 6 | 4,5 | 5 |
| 2 | Isooctylphenyl | 12,5 | | 6 | 4,5 | 5 |
| 3 | n-Dodecyl | 13,1 | (7) | 6 | 4,5 | 5 |
| 4 | " | 14,1 | (9) | 6 | 4,5 | 5 |
| 5 | — | — | — | — | 10,5 | — |
| 6 | Isotridecyl | 12 | | 10,5 | — | — |
| 7 | Isooctylphenyl | 12,5 | | 10,5 | — | — |
| 8 | n-Dodecyl- | 13,1 | (7) | 10,5 | — | — |
| 9 | " | 14,1 | (9) | 10,5 | — | — |
| 10 | Isotridecyl | 12 | | 6 | 4,6 | — |

0 041 704

Die Beispiele 3 bis 9 sind Vergleichsbeispiele und nicht erfindungsgemäß.

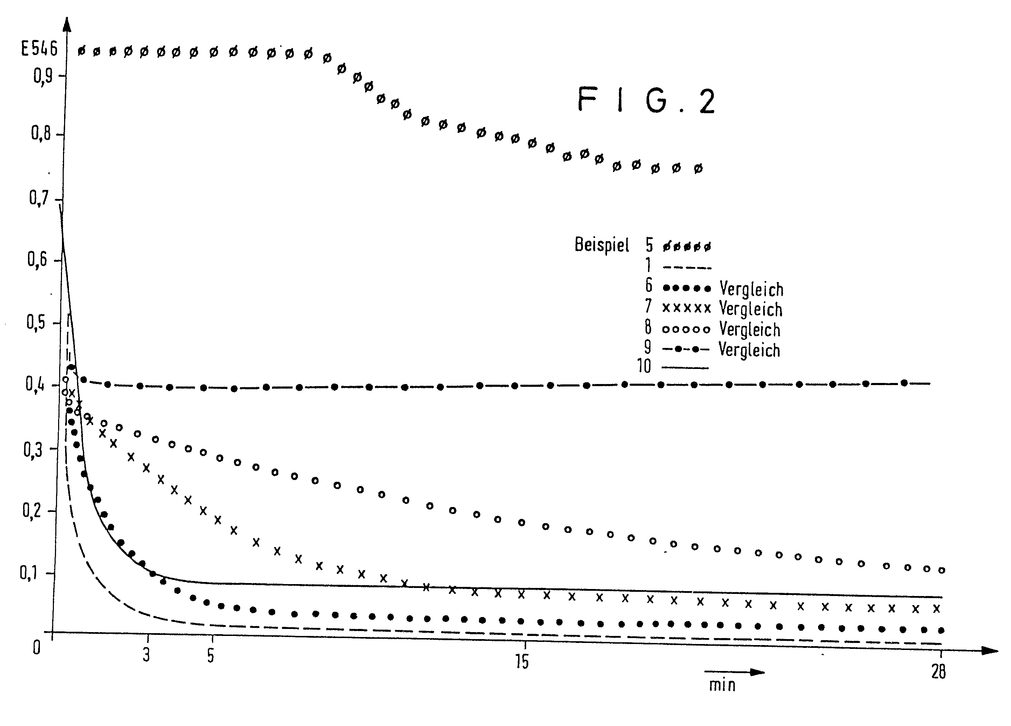

Die Ergebnisse sind in der beigefügten Zeichnung dargestellt. In dieser Fig. 1 eine graphische Darstellung der Änderung der Durchlässigkeit mit der Zeit für die Beispiele 1 bis 4, Fig. 2 eine entsprechende graphische Darstellung für die Beispiele 5 bis 10 und Beispiel 1.

Beispiel 11 (Anwendungsbeispiel)

Cholesterinbestimmung mit Cholesterinoxidase und Katalase (Katalase-Methode)

Bei dieser aus Z. Clin. Chem. Clin. Biochem. 12 (1974), 403 bekannten Methode wird das Probematerial mit einem Reagens versetzt, welches aus Cholesterinoxidase, Cholesterinesterase, Katalase, Acetylaceton, Methanol und Phosphatpuffer, pH 7,0 sowie Hydroxypolyäthoxydodekan besteht. Ein Nachteil dieses Reagens besteht darin, daß in vielen Fällen durch Chylomikronen bedingte Trübungen in der Probe nicht beseitigt werden können und falsch zu niedrige Cholesterinwerte erhalten werden.

Das Hydroxypolyäthoxydodekan wurde daher durch eine gleiche Menge des Mittels von Beispiel 1 ersetzt. Auch bei trüben Seren wurden hierbei klare Lösungen erhalten und die gemessenen Cholesterinwerte wiesen keine Abweichung von den richtigen Werten auf.

**Ansprüche**

1. Verfahren zur Auflösung von Chylomikronen in wäßrigem Medium durch Zusatz eines oberflächenaktiven Polyäthylenglykol-Derivats, dadurch gekennzeichnet, daß man dem wäßrigen Medium

a) 3-20 Vol.% wenigstens eines Polyäthylenglykoläthers eines Alkanols oder Alkylarylalkohols mit verzweigter Alkankette und einem HLB-Wert von 12 bis 14 und

b) 1-10 Vol.% wenigstens eines sekundären Alkansulfonats mit 10-20 C-Atomen im Molekül, sowie gegebenenfalls

c) ein Alkali-p-toluolsulfonat zusetzt.

2. Verfahren nach Anspruch 1, dadurch gekennzeichnet, daß man

a) 4 bis 8 Gew.-Teile Polyäthylenglykoläther und

b) 6 bis 2 Gew.-Teile Alkansulfonat

zusetzt.

3. Verfahren nach Anspruch 1 oder 2, dadurch gekennzeichnet, daß man 5 bis 10 Gew.-% Alkali-p-toluolsulfonat, bezogen auf die Summe von a) und b), zusetzt.

4. Verfahren nach einem der Ansprüche 1 bis 3, dadurch gekennzeichnet, daß man einen Polyäthylenglykoläther mit einem HLB-Wert von 12,5 bis 13,5 verwendet.

5. Verfahren nach einem der Ansprüche 1 bis 4, dadurch gekennzeichnet, daß man ein Alkansulfonat mit 13 bis 15 C-Atomen im Molekül verwendet.

6. Verfahren nach einem der Ansprüche 1 bis 5, dadurch gekennzeichnet, daß man ein Gemisch von sekundären Alkyl- mono- und disulfonaten verwendet.

7. Mittel zur Auflösung von Chylomikronen in wäßrigem Medium mit einem Gehalt an oberflächenaktivem Polyäthylenglykoläther, dadurch gekennzeichnet, daß es

a) 3-20 Vol.% wenigstens eines Polyäthylenglykoläthers eines Alkanols oder Alkylarylalkohols mit verzweigter Alkankette und einem HLB-Wert von 2 bis 14 und

b) 1-10 Vol.% wenigstens eines sekundären Alkansulfonats mit 10 bis 20 C-Atomen im Molekül sowie gegebenenfalls

c) ein Alkali-p-toluolsulfonat

enthält.

8. Mittel nach Anspruch 7, dadurch gekennzeichnet, daß es

a) 4 bis 8 Gew.-Teile Polyäthylenglykol und

b) 6 bis 2 Gew.-Teile Alkansulfonat

enthält.

9. Mittel nach Anspruch 7 oder 8, dadurch gekennzeichnet, daß es außerdem 5 bis 10 Gew.-% Alkali-p-toluolsulfonat enthält.

10. Mittel nach einem der Ansprüche 7 bis 9, dadurch gekennzeichnet, daß es einen Polyäthylenglykoläther mit einem HLB-Wert von 12,5 bis 13,5 enthält.

11. Mittel nach einem der Ansprüche 7 bis 10, dadurch gekennzeichnet, daß es ein Alkylsulfonat mit 13 bis 15 C-Atomen im Molekül enthält.

12. Mittel nach einem der Ansprüche 7 bis 11, dadurch gekennzeichnet, daß es ein Gemisch von sekundären Alkyl- mono- und disulfonaten enthält.

## Claims

1. Process for dissolving chylomicrons in an aqueous medium by adding a surface-active polyethylene glycol derivative, characterised in that one adds to the aqueous medium

a) 3-20 vol.% of at least one polyethylene glycol ether of an alkanol or alkylaryl alcohol with a branched alkane chain and with an HLB value of 12 to 14 and
b) 1-10 vol.% of at least one secondary alkane sulphonate with 10-20 C-atoms in the molecule, as well as optionally
c) an alkali metal p-toluenesulphonate.

2. Process according to claim 1, characterised in that one adds

a) 4 to 8 parts by weight of polyethylene glycol ether and
b) 6 to 2 parts by weight of alkane sulphonate.

3. Process according to claim 1 or 2, characterised in that one adds 5 to 10 wt.% of alkali metal p-toluenesulphonate, referred to the sum of a) and b).

4. Process according to one of claims 1 to 3, characterised in that one uses a polyethylene glycol ether with an HLB value of 12.5 to 13.5.

5. Process according to one of claims 1 to 4, characterised in that one uses an alkyl sulphonate containing 13 to 15 C-atoms in the molecule.

6. Process according to one of claims 1 to 5, characterised in that one uses a mixture of secondary alkyl mono- and di-sulphonates.

7. Agent for dissolving chylomicrons in an aqueous medium with a content of surface-active polyethylene glycol derivatives, characterised in that in contains

a) 3-20 vol.% of at least one polyethylene glycol ether of an alkanol or arylalkanol with a branched alkane chain and an HBL value of 12 to 14 and
b) 1-10 vol.% of at least one secondary alkane sulphonate with 10 to 20 C-atoms in the molecule, as well as optionally
c) an alkali metal-p-toluenesulphonate.

8. Agent according to claim 7, characterised in that it contains

a) 4 to 8 parts by weight of polyethylene glycol ether and
b) 6 to 2 parts by weight of alkane sulphonate.

9. Agent according to claim 7 or 8, characterised in that it also contains 5 to 10 wt.% of alkali metal p-toluenesulphonate.

10. Agent according to one of claims 7 to 9, characterised in that it contains a polyethylene glycol ether with an HLB value of 12.5 to 13.5.

11. Agent according to one of claims 7 to 10, characterised in that it contains an alkyl sulphonate with 13 to 15 C-atoms in the molecule.

12. Agent according to one of claims 4 to 11, characterised in that it contains a mixture of secondary alkyl mono- and disulphonates.

## Revendications

1. Procédé pour la dissolution de chylomicrons en milieu aqueux par addition d'un dérivé tensio-actif du polyéthylèneglycol, caractérisé en ce qu'on ajoute au milieu aqueux

a) 3-20 % en volume d'au moins un éther polyéthylèneglycolique d'un alcanol ou d'un alcool alcoylarylique avec une chaîne alcane ramifiée et une valeur de HLB de 12 à 14 et
b) 1-10 % en volume d'au moins un alcanesulfonate secondaire ayant 10-20 atomes de C dans la molécule, ainsi que le cas échéant
c) un p-toluènesulfonate alcalin.

2. Procédé suivant la revendication 1, caractérisé en ce qu'on ajoute

a) 4 à 8 parties en poids d'éther polyéthylèneglycolique et
b) 6 à 2 parties en poids d'alcanesulfonate.

3. Procédé suivant la revendication 1 ou 2, caractérisé en ce qu'on ajoute 5 à 10 % en poids de p-toluènesulfonate alcalin, par rapport à la somme de a) et b).

4. Procédé suivant l'une des revendications 1 à 3, caractérisé en ce qu'on utilise un éther polyéthylèneglycolique ayant une valeur de HLB de 12,5 à 13,5.

5. Procédé suivant l'une des revendications 1 à 4, caractérisé en ce qu'on utilise un alcanesulfonate ayant 13 à 15 atomes de C dans la molécule.

6. Procédé suivant l'une des revendications 1 à 5, caractérisé en ce qu'on utilise un mélange de mono- et disulfonates d'alcoyle secondaires.

7. Agent pour la dissolution de chylomicrons en milieu aqueux, ayant une teneur en éther polyéthylèneglycolique tensio-actif, caractérisé en ce qu'il contient

a) 3-20 % en volume d'au moins un éther polyéthylèneglycolique d'un alcanol ou arylalcanol avec une chaîne alcane ramifiée et une valeur de HLB de 2 à 14 et
b) 1-10 % en volume d'au moins un alcanesulfonate secondaire avec 10 à 20 atomes de C dans la molécule, ainsi que le cas échéant
c) un p-toluènesulfonate alcalin.

8. Agent suivant la revendication 7, caractérisé en ce qu'il contient

a) 4 à 8 parties en poids de polyéthylèneglycol et
b) 6 à 2 parties en poids d'un alcanesulfonate.

9. Agent suivant la revendication 7 ou 8, caractérisé en ce qu'il contient en outre 5 à 10 % en poids de p-toluènesulfonate alcalin.

10. Agent suivant l'une des revendications 7 à 9, caractérisé en ce qu'il contient un éther polyéthylèneglycolique avec une valeur de HLB de 12,5 à 13,5.

11. Agent suivant l'une des revendications 7 à 10, caractérisé en ce qu'il contient un sulfonate d'alcoyle avec 13 à 15 atomes de C dans la molécule.

12. Agent suivant l'une des revendications 7 à 11, caractérisé en ce qu'il contient un mélange de mono- et de disulfonates d'alcoyle secondaires.

F I G . 2

Beispiel 5 ⌀⌀⌀⌀⌀
        1 ─ ─ ─ ─
        6 ●●●●● Vergleich
        7 ××××× Vergleich
        8 ooooo Vergleich
        9 ─•─•─ Vergleich
       10 ───────

0 041 704